(19) Europäisches Patentamt
European Patent Office
Office européen des brevets

(11) Publication number: 0 330 444 A2

(12) EUROPEAN PATENT APPLICATION

(21) Application number: 89301705.3

(22) Date of filing: 22.02.89

(51) Int. Cl.4: C 07 D 335/12
C 07 D 413/10

(30) Priority: 22.02.88 US 158412

(43) Date of publication of application: 30.08.89 Bulletin 89/35

(84) Designated Contracting States: BE CH DE FR GB LI NL SE

(71) Applicant: EASTMAN KODAK COMPANY (a New Jersey corporation)
343 State Street
Rochester New York 14650 (US)

(72) Inventor: Chen, Chin Hsin c/o EASTMAN KODAK CO.
Patent Department 343 State Street
Rochester New York 14650 (US)

Fox, John Leonard c/o EASTMAN KODAK CO.
Patent Department 343 State Street
Rochester New York 14650 (US)

(74) Representative: Davis, Ian Ellison et al
Kodak Limited Patent Department Headstone Drive
Harrow Middlesex HA1 4TY (GB)

# (54) Novel thiorhodamines and a novel method of preparation.

(57) There are disclosed novel thiorhodamines having the following structural formula:

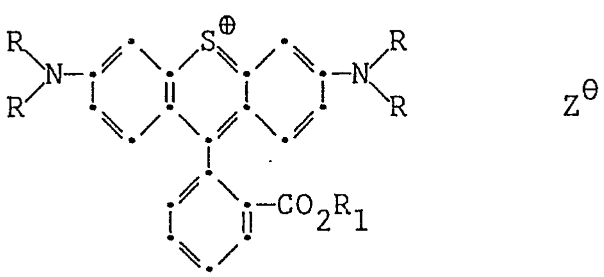

wherein
R and $R_1$ are individually H or alkyl of 1 to 5 carbon atoms, and
Z is an anion.

Also disclosed is the only known, and novel, method for making them, namely

a) conducting an addition reaction between a thioxanthone and a compound having the formula:

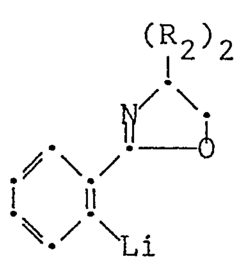

wherein $R_2$ is alkyl of 1 to 5 carbon atoms;

b) dehydrating the resulting compound of step a) in a strong acid to form a 9-[2-oxazolinylphenyl]thioxanthylium salt;

c) hydrolyzing the oxazoline ring with acid to the 9-(2-carboxyphenyl)thioxanthylium salt; and

d) optionally esterifying the resultant compound of step c) in an alkanol and strong acid, wherein the alkanol contains from 1 to 5 carbon atoms.

EP 0 330 444 A2

## Description

### NOVEL THIORHODAMINES AND A NOVEL METHOD OF PREPARATION

This invention relates to novel thiorhodamines and a method of preparing them from a thioxanthone.

Rhodamine 123, having the structure (1) below, is known as a chemotherapeutic agent:

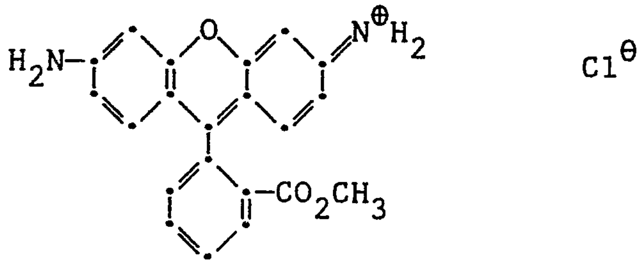

(This compound is conventionally synthesized by the following reaction:

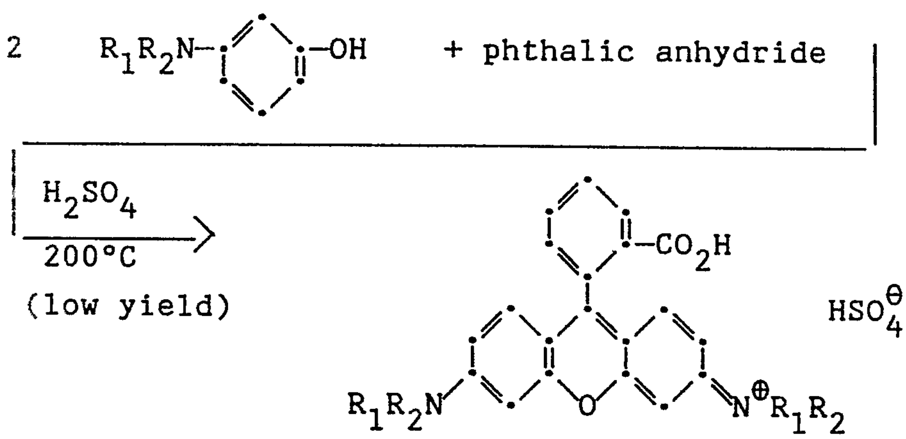


followed by optional esterification of the acid and extensive purification.) However, its use in vivo has required further activation such as by hyperthermia, or photosensitized energy, due to the lack of sufficient native or inherent cytotoxicity. The compound does, however, have excellent water solubility, pharmacokineticss, and biodistribution, and has been widely used as a laser dye along with the general class of rhodamines.

A problem addressed by this invention is the provision of a compound having the desirable water solubility of the rhodamines, along with enhanced cytotoxicity. Although there has been some evidence suggesting that thio analogs of oxa heterocycles have improved cytotoxicity, prior to this invention it has not been possible to make the thio analog of compound (1) using the conventional procedure shown in reaction (2).

In accord with one aspect of the invention, there is provided a thiorhodamine having the structural formula:

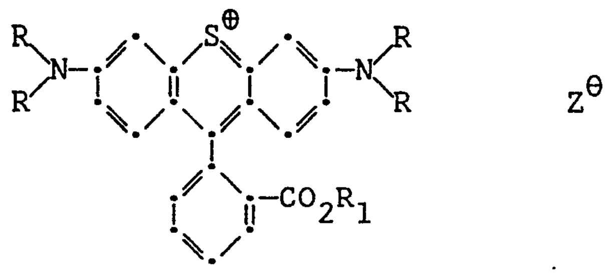

wherein
R and $R_1$ are individually H or alkyl of 1 to 5 carbon atoms, and
Z is an anion.

In accord with another aspect of the invention, there is provided a compound useful in forming a thiorhodamine, and having the structural formula:

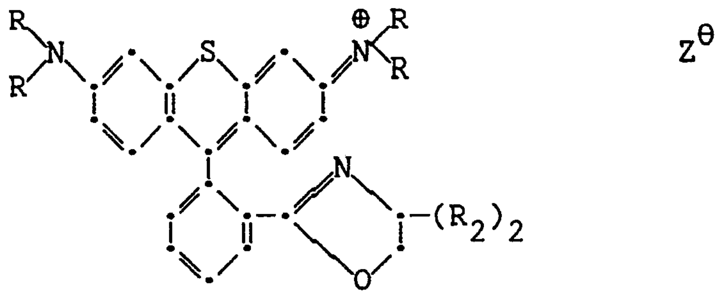

wherein
R is H or alkyl of 1 to 5 carbon atoms,
$R_2$ is alkyl of 1 to 5 carbon atoms, and
Z is an anion.

In accord with yet another aspect of the invention, there is provided a method for producing such a thiorhodamine from a thioxanthone having the structure:

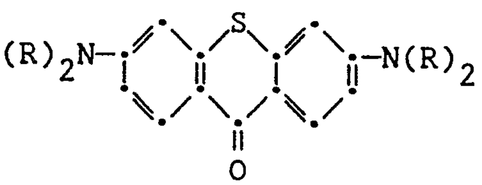

wherein R is as defined above. The method comprises the steps of

a) conducting an addition reaction between such thioxanthone and a compound having the formula:

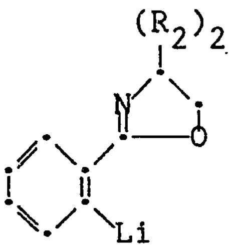

wherein $R_2$ is alkyl of 1 to 5 carbon atoms;

b) dehydrating the resultant compound of step a) in a strong acid to form a 9-(2-oxazolinylphenyl)thioxanthylium salt;

c) hydrolyzing the oxazoline ring with acid to the 9-(2-carboxyphenyl)thioxanthylium salt; and

d) optionally esterifying the resultant compound of step c) in an alkanol and strong acid, wherein the alkanol contains from 1 to 5 carbon atoms.

Thus it is an advantageous feature of the invention that a thiorhodamine compound is available with the water solubility of the rhodamine compounds, and the cytotoxicity of thio compounds.

It is another advantageous feature of the invention that novel fluorescent dyes are made available.

It is a related advantageous feature of the invention that a process is provided for making such compounds.

It has been discovered that novel thiorhodamines can be prepared having the structure set forth above. R and $R_1$ in such structure are individually H or alkyl of 1 to 5 carbon atoms. In the case of R, "alkyl" means unsubstituted straight or branched chain alkyl, e.g., methyl, ethyl, propyl, isopropyl, butyl, pentyl and the like. In the case of $R_1$, it is any of the unsubstituted choices for R, and also substituted alkyl, where the substituents are selected from aryl such as phenyl, to create, e.g., phenethyl, or benzyl.

A wide variety of anions is useful for $Z^{\theta}$. Examples include halide such as chloride, bromide, iodide, and fluoride; sulfonate including aliphatic and aromatic sulfonate such as methanesulfonate, trifluoromethanesulfonate, p-toluenesulfonate (tosylate), naphthalenesulfonate, and 2-hydroxyethanesulfonate; sulfamate such as cyclohexanesulfamate; sulfate such as methyl sulfate and ethyl sulfate; bisulfate; borate; tetrafluoroborate; perchlorate; nitrate; alkyl and dialkyl phosphate such as dimethyl phosphate, diethyl phosphate, and methyl hydrogen phosphate; pyrophosphate such as trimethylpyrophospate and diethyl hydrogen pyrophosphate; and carboxylate, preferably carboxy- and hydroxy-substituted carboxylate. Preferred examples of carboxylate include acetate, propionate, valerate, citrate, maleate, fumarate, lactate, succinate, tartrate and benzoate.

Because they impart to the dye greater water solubility than do the other anions, the most preferred anions are the halides and particularly chloride.

The most preferred example of such thiorhodamine compounds is 3,6-bis(dimethylamino)-9-(2-methoxycarbonylphenyl)thioxanthylium chloride.

These compounds are useful as fluorescent dyes, having a relative quantum yield, measured against Rhodamine 6G as a standard of about 50% .

In addition, the compounds have utility as chemotherapeutic agents, specifically in treating solid tumors, due to enhanced cytotoxicity. The improved or enhanced cytotoxicity, compared to Rhodamine 123, is apparent from <u>in vitro</u> tests.

The following is the procedure carried out in ascertaining the $IC_{50}$ concentrations of the thiorhodamines of this invention. ($IC_{50}$ is the in vitro molar concentration that inhibits 50% of tumor growth. The smaller the concentration, the higher the toxicity.):

For the $IC_{50}$ values for the human colon carcinoma and the human lung carcinoma lines, an adhering monolayer culture was used. Two days prior to plating, a number of cells obtained from the American Type Tissue Culture Collection was selected to grow as a stock, the number being that which would ensure the cells had not reached their plateau at the time of the in vitro test. On day 1 of the assay, asynchronous exponentially growing cells from this stock were suspended in a conventional complete medium comprising RPMI 1640 medium supplemented with 5% (v/v) fetal calf serum and 5% (v/v) NU serum obtained from Collaborative Research, and 20mM HEPES buffer, i.e., 4-(2-hydroxyethyl)-1-piperazineethanesulfonic acid. This was plated into a conventional 96-well tissue culture plate using a Cetus/Perkin Elmer automated pipetting device equipped with a dual supply manifold. The cells were then incubated for 48 hours at 37°C in a humidified atmosphere containing 5% $CO_2$. The cells were then incubated under red light for 3 hours in the presence of varying amounts of the compound of interest at 37°C in a humidified incubator containing 5% $CO_2$. Compounds were tested over a 6-log range of concentrations. After 3 hours the drug treated cells were then rinsed one time with complete medium as set forth above, and incubated in the complete medium the length of time appropriate to achieve four cell-doubling times (roughly 7 days for these cell lines). The medium was removed and the cells stained for 1-2 hours at 37°C with 0.1% (w/v) neutral red dye obtained from Eastman Kodak Co., dissolved in phosphate buffered saline (PBS) (without calcium or magnesium cations) at a pH of 6.85. The cells were rinsed twice with the same PBS and dried. Excess neutral red dye was eluted from each well with 50% (v/v) ethanol in 1% (v/v) glacial acetic acid. The absorbence of each well was quantitated using a Perkin-Elmer absorbence densitometer set at 540 nm. The % of inhibition of cell growth for a given concentration of compound was calculated using the equation:

$$F(x) = \%\ \text{inhibition} = \left[1 - \frac{At - Ab}{Au - Ab}\right] \times 100$$

where $A_t$ = absorbence of the cells treated with the candidate drug, $A_u$ = absorbence of the control treated with solvent only, and $A_b$ = absorbence of the media and well only (background). These results were plotted

as F(x) versus the log concentration of the test compound. The plotted data was then fitted to the curve for the function:

$F(x) = 100 - [100(X/A)^B/(1+(X/A)^B)]$

where x = the concentration of the test compound, A = the $IC_{50}$ value (50% inhibition on the plot) and B = the exponent of the fitted function.

Following the protocol, 3,6-bis(dimethylamino)-9-(2-methoxycarbonylphenyl)thioxanthylium chloride (Preparation E hereinafter) was tested against human lung adenocarcinoma and human colon carcinoma. As a control, Rhodamine 123 was also tested.

The $IC_{50}$ values were as follows:

Table 1 (of $IC_{50}$ Values)*

| Compound | A549 Human Lung Carcinoma | CX-1 Human Colon Carcinoma |
|---|---|---|
| Prep. 4 | 0.81 μMolar | 0.81 μMolar |
| Rhodamine 123 | >12.5 μMolar | >12.5 μMolar |

*Prep. 4 was not found to be superior to Rhodamine 123 in treating mouse leukemia. However, the apparent cancer utility is directed to solid tumors, rather than leukemia.

## Synthesis

Prior to this invention, there was no known method for making these compounds. That is, the process used to make compound (1) noted above could not be used to make the thio analog, any implication in U.S. Patent 3,687,678 to the contrary notwithstanding. That is, the above procedure (2) used to make Rhodamine 123, when applied to the thio analog of the starting material, fails because the mercapto analog of the starting phenol and phthalic anhydride will not produce thiorhodamine.

In accord with another aspect of the invention, a synthesis process has been discovered as set forth above. In that process, R is H or alkyl of 1 to 5 carbon atoms, and $R_2$ is alkyl of 1 to 5 carbon atoms. The alkyl of R and $R_2$ may be, for example, methyl, ethyl, propyl, isopropyl, butyl and pentyl. The strong acid of step b) is preferably fluoroboric acid, and of step c) is preferably HCl.

The following preparations are illustrative:

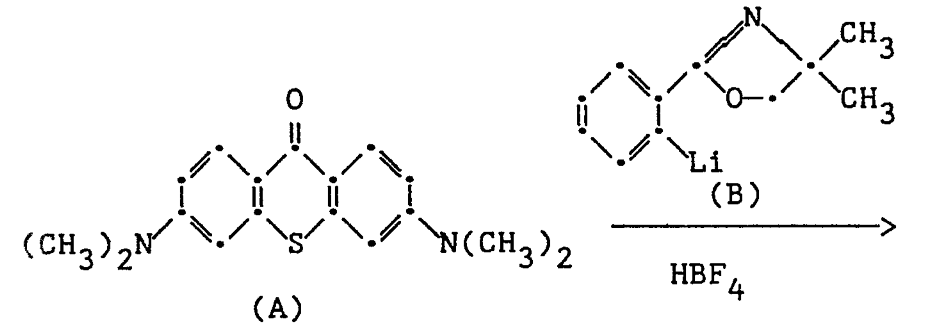


(A)

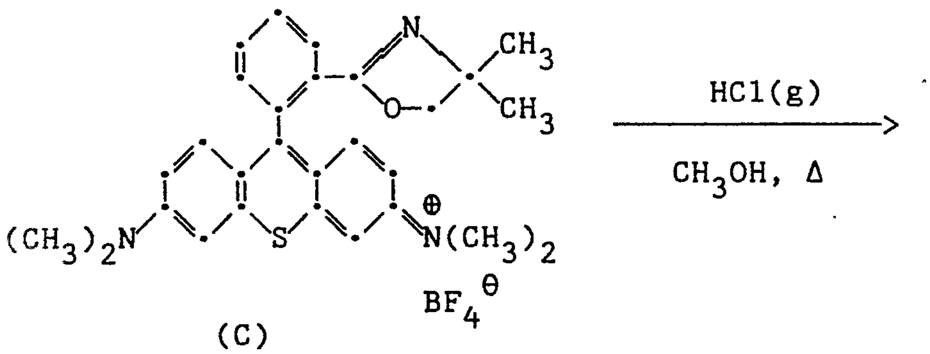


(C)

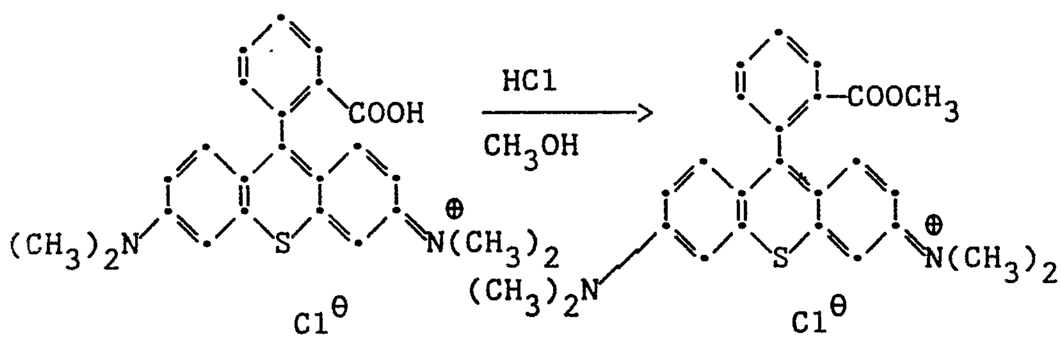


(D) (E)

Preparation 1: 3,6-Bis(dimethylamino)thioxanthone. (A)

This preparation is conventional. To a solution of 720 mg (2.5 mmol) of 3,6-Bis(dimethylamino)thioxanthene in 15 mL of acetone was added portionwise 1.19 g (7.5 mmol) of potassium permanganate. The mixture was allowed to stir overnight at room temperature. The solid manganese dioxide was filtered and washed with chloroform; the resulting filtrate yielded 220 mg of product. A Soxlet extraction of the manganese dioxide with chloroform yielded, upon cooling, an additional 440 mg of product. The combined fractions were flash chromatographed (24:1 $CH_2Cl_2$/EtOAc) to yield 550 mg (73%) of pure 3,6-Bis(dimethylamino)thioxanthone.

Preparation 2: 2-(4,4-dimethyl-2-oxazolin-2-yl)phenyl lithium. (B)

This was prepared, as is conventional, by the process of adding 7.4 ml of 1.7M (12.2 mmol) t-butyl lithium to a solution of, 1.55 g (6.1 mmol) of 2-(2-bromophenyl)-4,4-dimethyl-2-oxazoline in 75 ml of dry tetrahydrofuran (THF) at -78°C, at such a rate as to keep the internal reaction temperature below -70°C.

Example 1: 3,6-Bis(dimethylamino-9-[2-(4,4-dimethyl-2-oxazolin-2-yl)-phenyl]thioxanthylium tetrafluoroborate. (C)

The compound resulting from Preparation 2 was added to that of Preparation 1 by transferring Prep. 2's reaction mixture dropwise via cannula to a suspension of 1.4 g (4.7 mmol) of Prep. 1 in 50 ml of dry THF at -60°C. Following the addition, the cooling bath was removed and the reaction mixture allowed to warm to room temperature. After stirring for two hours to form a 9-(2-oxazolinylphenyl)thioxanthylium salt, 3 mL of 49% fluoroboric acid in water was added dropwise followed by rapid dropwise addition of 100 ml of anhydrous

diethyl ether. The solid was filtered and washed with ether to yield 2.38 g (93%). An analytical sample was purified by flash chromatography (10:1 $CH_2Cl_2$/MeOH with 0.5% of 49% fluoroboric acid).

This compound is novel. Other salts are obtainable by further ion exchange.

Example 2: 3,6-bis(dimethylamino)-9-(2-carboxyphenyl)thioxanthylium chloride. (D)

The reaction product (1 g) of Example 1 was refluxed in 100 mL of anhydrous methanol, and into this gaseous HCl was periodically bubbled over a 24 hour period to hydrolyze the oxazoline ring formed in Prep. 3 above. The solution was externally cooled to 0°C and slowly poured into a vigorously stirred 20% NaOH solution. The resulting precipitate was filtered, washed with water and air dried to give 0.68 g of the free acid form of the desired compound. An analytical sample was obtained by flash chromatography: mp 268-9°C; FD m/e 402 ($M^+$); $^1H$ NMR ($CDCl_3$) $\delta$ 2.98 (s, 12H, NMe), 6.48 (dd, J = 2.6, 8.9 Hz, 2H), 6.76 (d, J = 2.5 Hz, 2H), 6.88 (d, J = 8.9 Hz, 2H), 7.59 (m, 3H), 8.0 (d, J=7.4 Hz, 1H).

Example 3: 3,6-bis(dimethylamino)-9-(2-methoxycarbonylphenyl)thioxanthylium chloride. (E)

Next, 80 mg of the carboxylate (D) (Example 2) was refluxed in 5 mL of 5% HCl in methanol for 18 h. After removal of methanolic HCl under vacuum, the residue was dissolved in dichloromethane and flash chromatographed through a 6" x 1/2" silica gel (32-60 micron size) column first using acetonitrile and methanol to remove unesterified starting material. The product was then eluted with 100 mL of acetonitrile: methanol (1:1 v/v) containing 4 drops of conc. HCl to give 85 mg of the pure thiorhodamine (E). F.D. m/e 417 ($M^+$); $^1H$ NMR ($CDCL_3$/TMS) $\delta$.3.31 (s, 12H, NMe), 3.59 (s, 3H, $CO_2Me$), 6.93 (d, J = 9.4 Hz, 2H), (7.16 (d, J = 9.4 Hz, 2H), 7.29 (s, 2H), 7.7 (m, 3H), 8.29 (d, J = 7.4 Hz, 1H).

Example 4: Use of 3,6-Bis(dimethylamino-9-[2-(4,4-dimethyl-2-oxazolin-2-yl)-phenyl]thioxanthylium tetrafluoroborate

This was found to have the following dye properties:

| Absorption (EtOH) | | Fluorescence (EtOH) | |
|---|---|---|---|
| λmax | Extinction (ε) | Excitation | Emission Max |
| 572 nm | 67,200 | 571 nm | 596 nm |

Its relative quantium yield is as noted above (specifically, 49%), using a SPEX Fluorolog II spectrophotometer.

Example 5: Use of 3,6-bis(dimethylamino)-9-(2-methoxycarbonyphenyl)thioxanthylium chloride

This was found to have the following dye properties:

| Absorption (EtOH) | | Fluorescence (EtOH) | |
|---|---|---|---|
| λmax | Extinction (ε) | Excitation | Emission Max |
| 563 nm | 24,540 | 562 nm | 590 nm |

Its relative quantium yield was also 49%.

## Claims

1. A thiorhodamine having the following structural formula:

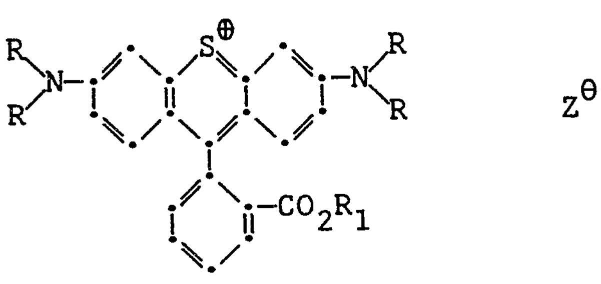

whereinR and $R_1$ are individually H or alkyl of 1 to 5 carbon atoms, and
Z is an anion.

2. A salt of the cation 3,6-bis(dimethylamino)-9-(2-methoxycarbonylphenyl)thioxanthylium.

3. A method for producing a thiorhodamine as defined in Claim 1 from a thioxanthone having the structure:

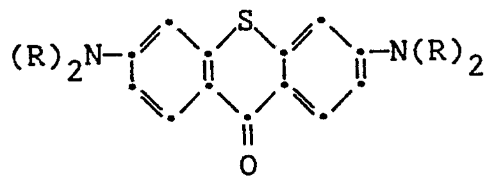

wherein R is as defined above, comprising the steps of:

a) conducting an addition reaction between such thioxanthone and a compound having the formula:

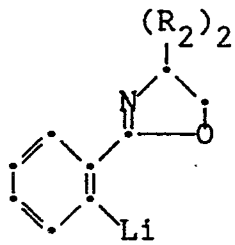

wherein $R_2$ is alkyl of 1 to 5 carbon atoms;

b) dehydrating the resulting compound of step a) in a strong acid to form a 9-(2-oxazolinylphenyl)thioxanthylium salt; and

c) hydrolyzing the oxazoline ring with acid to the 9-(2-carboxyphenyl)thioxanthylium salt.

4. A method as defined in claim 3, and further including the step of d) esterifying the resultant compound of step c) in an alkanol and strong acid, wherein the alkanol contains from 1 to 5 carbon atoms.

5. A method as defined in Claim 3, wherein R and $R_1$ are methyl, and the anion of the salt is $Cl^{\ominus}$.

6. A compound useful in forming a thiorhodamine, said compound having the structural formula:

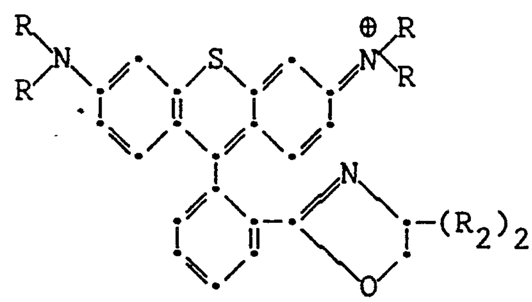

$Z^{\ominus}$

wherein

R is H or alkyl of 1 to 5 carbon atoms,

$R_2$ is alkyl of 1 to 5 carbon atoms, and

Z is an anion.

7. A salt of the cation 3,6-bis(dimethylamino)-9-[2-(4,4-dimethyl-2-oxazolin-2-yl)phenyl]thioxanthylium.

8. A salt as defined in claim 7 comprising 3,6-Bis(dimethylamino-9-[2-(4,4-dimethyl-2-oxazolin-2-yl)-phenyl]thioxanthylium tetrafluroborate.